Europäisches Patentamt

European Patent Office

Office européen des brevets

(11) Veröffentlichungsnummer: **0 028 627**
**B1**

(12) # EUROPÄISCHE PATENTSCHRIFT

(45) Veröffentlichungstag der Patentschrift:
**09.05.84**

(51) Int. Cl.³: **C 07 C 103/52, A 61 K 37/02**

(21) Anmeldenummer: **80901003.6**

(22) Anmeldetag: **14.05.80**

(86) Internationale Anmeldenummer:
**PCT/DE 80/00067**

(87) Internationale Veröffentlichungsnummer:
**WO 80/02560 (27.11.80 Gazette 80/27)**

(54) **VERFAHREN ZUR HERSTELLUNG VON THYMOSIN-ALPHA1 UND DERIVATEN DAVON.**

(30) Priorität: **15.05.79 DE 2919592**

(43) Veröffentlichungstag der Anmeldung:
**20.05.81 Patentblatt 81/20**

(45) Bekanntmachung des Hinweises auf die Patenterteilung:
**09.05.84 Patentblatt 84/19**

(84) Benannte Vertragsstaaten:
**FR**

(56) Entgegenhaltungen:
**US - A - 4 116 951**

**Angewandte Chemie, Int. Ed. Band 18, No. 5, Mai 1979
(Weinheim DT), Ch. Birr und U. Stollenwerk: "Synthesis
of Thymosin alpha1, a polypeptide of the Thymus" Seiten
394-395, siehe das ganze Dokument, übereinstimmend
mit Angewandte Chemie, Band 92, Seite 422, 1979
Chemical Abstracts, Band 91, No. 7, 13. August 1979
(Columbus, Ohio, USA) Ch. Birr et al.: "Preparative
merits of the mixed anhydride (MA) method in the excess
use of D d Z - amino - acids in the peptide synthesis of
biologically active new antamanide analogs", siehe
Seite 758, Spalte 1, Zusammenfassung Nr. 57489z. Int. J.
Pept. Protein. Res. 1979, 13(3) 287-295**

(73) Patentinhaber: **Max-Planck-Gesellschaft zur Förderung
der Wissenschaften e.V., Bunsenstrasse 10,
D-3400 Göttingen (DE)**

(72) Erfinder: **BIRR, Christian, Eichendorffstrasse 31,
D-6906 Leimen/St. Ilgen (DE)**
Erfinder: **STOLLENWERK, Ulrich, Kastellweg 17,
D-6900 Heidelberg (DE)**

(74) Vertreter: **Huber, Bernhard, Dipl.-Chem. et al,
Patentanwälte Dipl.-Ing. H.Weickmann Dipl.-Phys.Dr.
K.Fincke Dipl.-Ing. F.A.Weickmann Dipl.-Chem. B. Huber
Dr.-Ing. H. Liska Dipl.-Phys.Dr. J. Prechtel
Postfach 860820, D-8000 München 86 (DE)**

**Beschreibung**

Die Erfindung betrifft ein Verfahren zur Herstellung von Thymosin-$\alpha_1$ und bestimmten Derivaten desselben aus Peptidfragmenten.

Thymosin-$\alpha_1$ ist ein besonders saures Polypeptid der Thymusdrüse, welches die Sequenz Ser-Asp--Ala-Ala-Val-Asp-Thr-Ser-Ser-Glu-Ile-Thr-Thr-Lys--Asp-Leu-Lys-Glu-Lys-Lys-Glu-Val-Val-Glu-Glu-Ala--Glu-Asn besitzt und in dem die Aminosäure Nr. 1, Serin, acetyliert vorliegt. Beim Thymosin-$\alpha_1$ wurden interessante biologische Eigenschaften gefunden, welche seine Brauchbarkeit bei der Krebsbekämpfung und im Rahmen der Regulation des immunologischen Abwehrapparates erwarten lassen [Cancer Treatment Reports, Vol. 62, Nr. 11 (1978)]. So wurde beispielsweise gefunden, dass die durch Bestrahlung im Rahmen der Krebstherapie resultierende Immunsuppression durch Behandlung mit Thymosin-$\alpha_1$ vermindert werden kann.

Es besteht daher Interesse an einem Verfahren zur synthetischen Herstellung von Thymosin-$\alpha_1$.

Die chemische Totalsynthese von Thymosin-$\alpha_1$ ist bereits bekannt aus J. A. C. S. 101, 1, 253-254 (1979). Bei diesem Verfahren werden nach den bisher üblichen Methoden der Peptidsynthese zuerst mehrere verschiedene Peptidfragmente synthetisiert, die schliesslich nach verschiedenen Methoden, vor allem der Azidmethode, miteinander kondensiert werden. Das bekannte Verfahren weist jedoch den Nachteil auf, sehr aufwendig zu sein und trotzdem nur sehr geringe Ausbeuten zu liefern.

Auch aus US-PS 4 116 951 ist eine Totalsynthese von Thymosin-$\alpha_1$ bekannt, die über sehr viele Einzelschritte mit aufwendiger Aufarbeitung erfolgt.

Der Erfindung liegt die Aufgabe zugrunde, ein Verfahren zur Synthese von Thymosin-$\alpha_1$ zu schaffen, welches diese Nachteile nicht aufweist und in einfacher Reaktion mit guter Ausbeute zum gewünschten Produkt führt. Das Verfahren soll sich ausserdem dazu eignen, ohne wesentliche Änderungen auch die Herstellung von biologisch interessanten Derivaten und Analogen des Thymosin-$\alpha_1$ zu ermöglichen.

Gelöst wird diese Aufgabe erfindungsgemäss durch ein Verfahren zur Herstellung von Thymosin-$\alpha_1$ oder einem Derivat davon, bei welchem wenigstens eine der Aminosäuren 10, 15, 21, 25 und 28 als Amid oder Alkylamid vorliegt oder/und die Acetylgruppe durch eine andere Acylgruppe ersetzt ist, durch Herstellung einer Reihe von schutzgruppenhaltigen Peptidfragmenten, Kondensationen derselben und nachfolgende Abspaltung der Schutzgruppen, welches dadurch gekennzeichnet ist, dass man

a) das N-terminal ungeschützte, C-terminal veresterte oder trägergebundene C-terminale Peptidfragment in wenigstens 1,5fachem stöchiometrischem Unterschuss mit dem benachbarten N-terminal Ddz-geschützten, C-terminal ungeschützten Peptidfragment in wasserfreiem organischem Lösungsmittel mit Dicyclohexylcarbodiimid und 1-Hydroxybenzotriazol (DDC/1-HOBt) kondensiert,

b) durch Zusatz von Trifluoressigsäure in geringem stöchiometrischem Überschuss die Ddz-Gruppe des kondensierten Fragments abspaltet,

c) überschüssige Säure mit einer organischen Base neutralisiert, und

d) das so erhaltene N-terminal ungeschützte verlängerte C-terminale Fragment mit dem jeweils nächsten N-terminal Ddz-geschützten Fragment unter Wiederholung der Schritte a), b) und c) so oft kondensiert, bis die Peptidkette vollständig ist und schliesslich die verbleibenden Schutzgruppen in üblicher Weise abspaltet, wobei als Seitenschutzgruppen für Asp und Glu t-Butylestergruppen, für Ser und Thr tert.-Butylgruppen, für Lys Benzyloxycarbonylgruppen und für Asn 4,4'-Dimethoxybenzhydrylgruppen verwendet werden, und gegebenenfalls die N-terminale Acetylgruppe gegen eine andere Acylgruppe ausgetauscht wird.

Die hier verwendeten Abkürzungen entsprechen den IUPAG-IUB-Vorschlägen [J. Biol. Chem. 247, 977-983 (1972)]. Ddz bedeutet $\alpha,\alpha$-Dimethyl-3,5--dimethoxybenzyloxycarbonyl, Z bedeutet Benzyloxycarbonyl, Ac bedeutet Acetyl, OBut bedeutet t-Butylester und Mbh bedeutet 4,4'-Dimethyloxybenzhydryl. Als Alkylgruppe kommen geradkettige oder verzweigte Reste mit 1 bis 6, vorzugsweise 1 bis 4 C-Atomen in Betracht. Als Träger werden die dem Fachmann bekannten Trägermaterialien, die für die Immobilisierung von Peptiden und Proteinen geeignet sind, verwendet.

Wesentlich beim erfindungsgemässen Verfahren ist es, die Fragmente, welche das N-terminale Endstück des Thymosin-$\alpha_1$-Moleküls darstellen, in einem wenigstens 1,5fachen, vorzugsweise 2- bis 2,5fachen stöchiometrischen Überschuss einzusetzen. Da bei der Kondensation von zwei Fragmenten jeweils ein neues grösseres C-terminales Fragment mit einer N-terminalen Ddz-Schutzgruppe entsteht und letztere für die nächste Kondensationsstufe mit einer sehr kleinen Menge Säure bereits abgespalten werden kann, ist auch zur Neutralisation nur eine kleine Menge Base erforderlich. Damit werden die bei den bisher bekannten Verfahren üblichen vielen Einzelschritte und Zwischenaufarbeitungen dahingehend vereinfacht, dass zur Isolierung des jeweiligen Reaktionsprodukts eine einmalige chromatographische Trennung des Reaktionsgemisches an einer Säule angewendet wird. Bevorzugt verwendet man Sephadex LH 20 im Lösungsmittel, Methanol und 2,2,2-Trifluoräthanol. Im Falle der trägergebundenen Fragmentkondensation erübrigt sich auch die letztgenannte chromatographische Zwischenproduktisolierung.

Da erfindungsgemäss in wasserfreiem organischem Lösungsmittel gearbeitet wird, werden besondere Trocknungsschritte zur Entfernung von Wasser überflüssig. Auch die zweckmässig durch Chromatographie durchgeführte Reinigung des vollgeschützten Endprodukts kann im gleichen wasserfreien organischen Lösungsmittel erfolgen.

Zu Schritt a) wird als Lösungsmittel vorzugsweise Dimethylformamid verwendet. Andere geeignete Lösungsmittel sind N-Methylpyrrolidon und Mischungen aus beiden sowie Dimethylacetamid.

In Schritt b) wird als Lösungsmittel für die Säure Dichlormethan bevorzugt. Andere geeignete Lösungsmittel sind Chloroform, Tetrahydrofuran und Dioxan.

Besonders bevorzugt wird 1- bis etwa 5%ige Trifluoressigsäurelösung in Dichlormethan verwendet. Ein besonderer Vorzug der Trifluoressigsäure besteht darin, dass sie auch im Rahmen der abschliessenden Abspaltung der tert.-Butylesterschutzgruppen und der 4,4'-Dimethoxybenzhydrylschutzgruppe verwendet werden kann, wobei lediglich ihre Konzentration erhöht wird.

Zur Neutralisation können grundsätzlich alle im organischen Lösungsmittel löslichen oder trägergebundenen, organischen tertiären Amine verwendet werden. Besonders bevorzugt wird N-Methylmorpholin, da es Racemisierungen unterdrückt und damit die optische Reinheit des Produkts verbessert.

Nach Beendigung von Schritt a) zieht man das Lösungsmittel zweckmässig im Vakuum ab und reinigt den Rückstand chromatographisch, beispielsweise über ein Molekularsiebmaterial, wie vernetztes Dextran, Polystyrol oder dergleichen. Die Chromatographie erfolgt in einem geeigneten wasserfreien Lösungsmittel, wie einem Alkanol, halogeniertem Kohlenwasserstoff usw. Bevorzugt wird Methanol. Im Falle der trägergebundenen Fragmentkondensation wird lediglich mit Dimethylformamid und Dichlormethan der polymere Träger gewaschen.

Die als Ausgangsprodukte für das erfindungsgemässe Verfahren verwendeten Peptidfragmente lassen sich nach den bekannten Methoden der Peptidsynthese herstellen. Vorzugsweise erfolgt ihre Synthese ebenfalls unter Anwendung von Ddz-Schutzgruppen. Dabei werden gemischte Anhydride der Ddz-Aminosäuren mit Isobutyloxycarbonylchlorid/N-Methylmorpholin durch N-terminale schrittweise Sequenzverlängerung aufgebaut. Besonders bevorzugt werden 1,5 bis 2 Äquivalente der jeweiligen Ddz-Aminosäure, N-Methylmorpholin und sek.-Butyloxycarbonylchlorid in Dichlormethan gelöst und bei etwa —10 bis —20°C mit einer Lösung gemischt, welche man durch Umsetzung eines äquivalentes Ddz-Aminosäure-tert.-butylester bzw. Ddz-Oligopeptid-tert.-butylester in Dichlormethan, welches 1 bis 5% Trifluoressigsäure enthält und etwa eine viertel bis eine halbe Stunde bei Normaltemperatur gehalten und dann mit N-Methylmorpholin neutralisiert wird, erhält. Die gemischten Lösungen werden dann bei Zimmertemperatur etwa 30 Minuten bis 2 Stunden reagieren gelassen. Das erhaltene Reaktionsgemisch wird zur Trockne im Vakuum eingedampft und chromatographisch über einem geeigneten Chromatographiematerial, wie z.B. vernetztes Dextran, wie Sephadex LH 20, in einem Lösungsmittel, wie Methanol, gereinigt. Nach Entfernung dieses Lösungsmittels kann unmittelbar die nächste Kondensationsstufe in der beschriebenen Weise folgen, bis das jeweilige Peptidfragment fertiggestellt ist. Im Falle der trägergebundenen Fragmentkondensation wird nach erfolgter Reaktion mit Dimethylformamid und Dichlormethan der polymere Träger gewaschen.

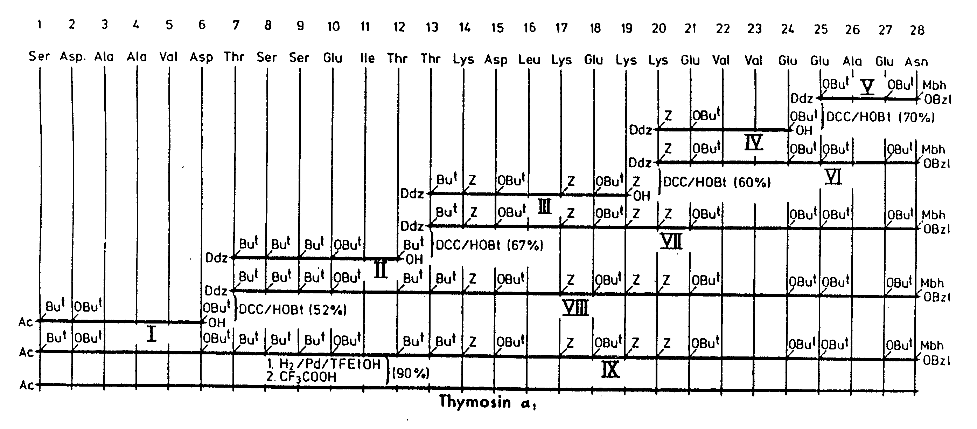

Der Aufbau des Thymosin-$\alpha_1$ aus den so erhaltenen Fragmenten wird durch die beigefügte Zeichnung weiter veranschaulicht. Man erkennt, dass fünf Fragmente hergestellt werden, welche aus den Aminosäuren 1 bis 6 (Fragment I), 7 bis 12 (Fragment II), 13 bis 19 (Fragment III), 20 bis 24 (Fragment IV) sowie 25 bis 28 (Fragment V) bestehen. Fragment V als C-terminales Fragment wird dann erfindungsgemäss mit Fragment IV umgesetzt, wobei man das verlängerte C-terminale Fragment VI erhält. In gleicher Weise wird Fragment VI mit Fragment III erneut umgesetzt, wobei Fragment VII als neues C-terminales Fragment entsteht. Letzteres wird mit Fragment II unter Bildung von Fragment VIII und dieses schliesslich mit Fragment I unter Bildung der fertigen Peptidkette IX umgesetzt, aus welcher lediglich noch die Schutzgruppe abgespalten wird. Soll gegebenenfalls die Acetylgruppe gegen eine andere Acylgruppe ausgetauscht werden, so erfolgt dies beim Aufbau des Fragments I. Die Fragmente I und V werden vorzugsweise als Benzylester, V gegebenenfalls trägergebunden, II, III und IV als Methylester hergestellt. Die Freisetzung der Carboxylendgruppe aus dem Ester erfolgt zweckmässig durch alkalische Verseifung, im Falle des Methylesters in wässrigem Dioxan, im Falle des Benzylesters in Tetrahydrofuran/Methanol.

Die Kondensationsreaktion selbst wird vorzugsweise in Dimethylformamid als Lösungsmittel durchgeführt. Die Ausbeuten in den einzelnen Kondensationsschritten sind dabei ausgezeichnet und erreichen bis 90%. Bei Fragment I wird die Benzylestergruppe zweckmässig abhydriert. Ein geeignetes Lösungsmittel ist eine Mischung von Propanol und Eisessig sowie 2,2,2-Trifluoräthanol. Als Katalysator wird zweckmässig Palladium-auf-Kohle, Palladium- oder Platinmoor verwendet.

Wie bereits erwähnt, gestattet das erfindungsgemässe Verfahren auch die Synthese von bestimmten Thymosin-$\alpha_1$-Derivaten, und zwar solchen, bei denen die Aminosäuren 10 (Glu), 15 (Asp), 21 (Glu), 25 (Glu) und 28 (Asn) anstelle einer Carboxylgruppe eine Amid- oder Alkylamidgruppe aufweisen. In diesem Falle werden in der entsprechenden Synthesestufe des Fragments anstelle der im natürlichen Thymosin-$\alpha_1$ vorliegenden jeweiligen Aminosäure die bereits in das Amid oder Alkylamid überführten Derivate dieser Aminosäure eingesetzt. Bei Asn wird dabei das entsprechende Diamid verwendet, da Asn an sich bereits ein Amid darstellt.

Die genannten Derivate besitzen besonders therapeutisches Interesse und weisen eine gegenüber dem Thymosin-$\alpha_1$ veränderte Wirksamkeit auf. Als neue Verbindungen stellen diese Analogen ebenfalls einen Gegenstand der Erfindung dar.

Auch die N-terminale Acetylgruppe kann zur Änderung der Wirkung bzw. Wirkungsstärke gegen eine andere Acylgruppe ausgetauscht werden. Vorzugsweise wird dabei anstelle des Acetylrests ein Acylglycinrest eingeführt.

Das folgende Beispiel erläutert die Erfindung weiter unter Bezugnahme auf die beigefügte Zeichnung.

*Beispiel*

*Herstellung von Thymosin-$\alpha_1$*

Die Fragmente I (1 bis 6), II (7 bis 12), III (13 bis 19), IV (20 bis 24) und V (25 bis 28) wurden in Lösung unter Verwendung überschüssiger gemischter Anhydride der Ddz-Aminosäuren mit Isobutyloxycarbonylchlorid/N-Methylmorpholin durch N-terminale, schrittweise Sequenzverlängerung allseits geschützt aufgebaut. Danach lagen die Fragmente I

und V als Benzylester, II, III und IV als Methylester mit folgenden über alle Schritte berechneten Ausbeuten vor:

I (67%), II (20%), III (31%), IV (56%) und V (39%).

Nach alkalischer Verseifung in wässrigem Dioxan wurde Fragment IV in Dimethylformamid mit V kondensiert, nachdem aus letzterem vorher die Ddz-Gruppe mit 1%iger Trifluoressigsäure in Dichlormethan (V/V) abgespalten worden war. Die Kondensation erfolgte mit Dicyclohexylcarbodiimid/1-Hydroxybenzotriazol (DCC/HOBt) in 24 Stunden bei 20°C mit 70% Ausbeute. Man erhält so Fragment VI.

Fragment III wird nach alkalischer Verseifung in wässrigem Tetrahydrofuran/Methanol mit Fragment VI unter den vorstehend beschriebenen Bedingungen in Dimethylformamid bei Zimmertemperatur in 66 Stunden verknüpft. Man erhält Fragment VII mit 60% Ausbeute.

In der nächsten Stufe wird Fragment VII nach in gleicher weise wie bei V erfolgter Ddz-Abspaltung in Dimethylformamid N-terminal mit Fragment II verknüpft, welches zuvor in Dioxan/Wasser, 8:2 (V/V) verseift worden war. Die Kondensation wurde 18 Stunden bei 0°C und 10 Stunden bei 20°C durchgeführt. Ausbeute 67% an Fragment VIII.

Vor der letzten Kondensation wurde vom N-terminalen Acetylfragment I die Benzylestergruppe hydrogenolytisch in Propanol/Eisessig abgespalten. Die freie Säure von I wurde in N-Methylpyrrolidon/Dimethylformamid 2:1 (V/V) in 24 Stunden mit VIII nach vorheriger Ddz-Abspalung umgesetzt. Ausbeute an Produkt IX 52%.

IX wurde in 2,2,2-Trifluoräthanol über Sephadex LH 20 chromatographisch gereinigt. Aminosäure-Analyse (berechnete Werte in Klammern: 6 N HCl 110°C, 24 Stunden); Asp 4,11(4); Thr 2,64(3); Ser 2,38(3); Glu 6,80(6); Lys 4,59(4); Ile 1,13(1); Leu 1,23(1); Ala 2,51(3); Val 2,41(3).

In drei abschliessenden Reaktionsschritten wurde IX von allen Schutzgruppen befreit. Durch Hydrogenolyse in 2,2,2-Trifluoräthanol mit Pd/C liessen sich alle Benzyloxycarbonylschutzgruppen und der C-terminale Benzylester entfernen (Ausbeute 96%). Durch 30 Minuten Einwirkung von Trifluoressigsäure/Dichlormethan 1:1 (V/V) in Gegenwart von 10 Vol.-% Anisol wurden hauptsächlich die tert.-Butylestergruppen abgespalten. Nach Abziehen der flüchtigen Bestandteile bei Raumtemperatur im Vakuum versetzte man den Polypeptid/Anisol-Rückstand mit reiner Trifluoressigsäure, um die 4,4'-Dimethoxybenzhydrylschutzgruppe und verbliebene tert.-Butylreste abzuspalten (120 Minuten, ca. 20°C). Nach Fällen und Waschen mit Äther wurde das synthetische Thymosin-$\alpha_1$ chromatographisch gereinigt. Dazu wurde an einer Säule (0,6 × 240 cm), die Bio-Gel P6 enthielt, in 1% Essigsäure (10% an Trifluoräthanol) zunächst das Retentionsvolumen für die oxidierte Insulin-B-Kette (MG: 3495) bestimmt und anschliessend das synthetische Thymosin-$\alpha_1$ chromatographiert. Es trat mit dem seinem Molekulargewicht (3107) entsprechenden Elutionsvolumen aus der Säule aus (Ausbeute 90%, bezogen auf IX). Aminosäuren-Analyse (berechnete Werte in Klammern: 6 N HCl/110°C/24, 48, 96 Stunden); Asp 4,11(4); Thr 2,86(3); Ser 2,70(3); Glu 5,89(6); Lys 3,98(4);

Ile 0,97(1); Leu 1,00(1); Ala 3,05(3); Val 2,97(3).

Dünnschichtchromatogramm (Kieselgel Merck 60 F-254; 0,25 mm). $R_f$ = 0,16 (n-Butanol/Pyridin/Eisessig/Wasser 5:5:1:4 (V/V), einheitlich.

$[\alpha]_\lambda^{25}$: —96° (579 nm).

—201,7° (435 nm), —242,5° (408 nm), —338,5° (365 nm), —587,0° (313 nm); c = 0,083 in Wasser.

Das synthetische Thymosin-$\alpha_1$ erwies sich im Test auf Lymphozytenstimulation, im E-Rosetten-Test und im Mitogen-Test als biologisch aktiv.

## Patentansprüche

1. Verfahren zur Herstellung von Thymosin-$\alpha_1$ oder einem Derivat davon, bei welchem weigstens eine der Aminosäuren 10, 15, 21, 25 und 28 als Amid oder Alkylamid vorliegt oder/und die Acetylgruppe durch eine andere Acylgruppe ersetzt ist, durch Herstellung einer Reihe von schutzgruppenhaltigen Peptidfragmenten, Kondensation derselben und nachfolgende Abspaltung der Schutzgruppen, dadurch gekennzeichnet, dass man

a) das N-terminal ungeschützte, C-terminal veresterte oder trägergebundene, C-terminale Peptidfragment in wenigstens 1,5fachem stöchiometrischem Unterschuss mit dem benachbarten N-terminal Ddz-geschützten, C-terminal ungeschützten Peptidfragment in wasserfreiem organischem Lösungsmittel mit Dicyclohexylcarbodiimid und 1-Hydroxybenzotriazol kondensiert,

b) durch Zusatz von Trifluoressigsäure in geringem stöchiometrischem Überschuss die Ddz-Gruppe des kondensierten Fragments abspaltet,

c) überschüssige Säure mit einer organischen Base neutralisiert, und

d) das so erhaltene N-terminal ungeschützte verlängerte C-terminale Fragment mit dem jeweils nächsten N-terminal Ddz-geschützten Fragment unter Wiederholung der Schritte a), b) und c) so oft kondensiert, bis die Peptidkette vollständig ist und schliesslich die verbleibenden Schutzgruppen in üblicher Weise abspaltet, wobei als Seitenschutzgruppen für Asp und Glu t-Butylestergruppen, für Ser und Thr tert.-Butylgruppen, für Lys Benzyloxycarbonylgruppen und für Asn 4,4'-Dimetoxybenzhydrylgruppen verwendet werden, und gegebenenfalls die N-terminale Acetylgruppe gegen eine andere Acylgruppe ausgetauscht wird.

2. Verfahren nach Anspruch 1, dadurch gekennzeichnet, dass Peptidfragmente verwendet werden, welche wenigstens einen der Glutaminsäureester 10, 21, 25 oder/und den Asparaginsäurerest 15 oder/und den Asparaginrest 28 in Form des Amids oder Alkylamids bzw. im Falle des Asparagins 28 des Diamids, enthalten.

3. Verfahren nach Anspruch 1 oder 2, dadurch gekennzeichnet, dass als Lösungsmittel in Schritt a) Dimethylformamid verwendet wird.

4. Verfahren nach einem der Ansprüche 1 bis 3, dadurch gekennzeichnet, dass in Schritt b) Trifluoressigsäure in Dichlormethan zugesetzt wird.

5. Verfahren nach Anspruch 4, dadurch gekennzeichnet, dass 1 bis 5%ige Lösung von Trifluoressigsäure verwendet wird.

6. Verfahren nach einem der vorhergehenden Ansprüche, dadurch gekennzeichnet, dass die Kondensation von Schritt a) bei einer Temperatur zwischen 0 und 30°C durchgeführt wird.

7. Verfahren nach einem der vorhergehenden Ansprüche, dadurch gekennzeichnet, dass die Benzyloxycarbonylschutzgruppen und eine C-terminale Benzylestergruppe durch Hydrogenolyse in Trifluoräthanol abgespalten werden.

8. Verfahren nach einem der vorhergehenden Ansprüche, dadurch gekennzeichnet, dass tert.-Butylestergruppen mit 40 bis 60%iger Trifluoressigsäure in Dichlormethan in Gegenwart von Anisol abgespalten werden.

9. Verfahren nach einem der vorhergehenden Ansprüche, dadurch gekennzeichnet, dass die 4,4'-Dimethylbenzhydrylschutzgruppe in reiner Trifluoressigsäure abgespalten wird.

10. Verfahren nach einem der vorhergehenden Ansprüche, dadurch gekennzeichnet, dass in Schritt b) mit N-Methylmorpholin neutralisiert wird.

11. Verfahren nach einem der vorhergehenden Ansprüche, dadurch gekennzeichnet, dass die Schritte a), b) und c) an den trägergebundenen Fragmenten V, VI, VII, VIII und IX ausgeführt werden.

12. Polypeptide der Sequenz Ser-Asp-Ala-Ala-Val-Asp-Thr-Ser-Ser-Glu-Ile-Thr-Thr-Lys-Asp-Leu-Lys-Glu-Lys-Lys-Glu-Val-Val-Glu-Glu-Ala-Glu-Asn, dadurch gekennzeichnet, dass wenigstens eine der Aminosäuren 10, 15, 21, 25 oder/und 28 als Amid oder Alkylamid vorliegt oder/und Aminosäure 1 eine von Acetyl verschiedene Acylgruppe mit bis zu 6 C-Atomen, insbesondere einen Acylglycinrest, trägt.

13. Thymosin-$\alpha_1$-Fragment I der Formel

$$\overset{1}{\text{Ac-Ser(Bu}^t)}\text{-}\overset{2}{\text{Asp(OBu}^t)}\text{-}\overset{3}{\text{Ala}}\text{-}\overset{4}{\text{Ala}}\text{-}\overset{5}{\text{Val}}\text{-}\overset{6}{\text{Asp((OBu}^t),}$$

wobei das Fragment anstelle der Acetylgruppe eine andere Acylgruppe, insbesondere eine Acylglycingruppe, tragen kann, sowie dessen Benzylester.

14. Thymosin-$\alpha_1$-Fragment II der Formel

$$\overset{7}{\text{Ddz-Thr(Bu}^t)}\text{-}\overset{8}{\text{Ser(Bu}^t)}\text{-}\overset{9}{\text{Ser(Bu}^t)}\text{-}\overset{10}{\text{Glu(OBu}^t)}\text{-}\overset{11}{\text{Ile}}\text{-}\overset{12}{\text{Thr}}\text{-}$$
$$\text{(OH)(Bu}^t),$$

wobei der Glutaminsäurerest 10 als Amid oder Alkylamid vorliegen kann, sowie dessen Methylester.

15. Thymosin-$\alpha_1$-Fragment III der Formel

$$\overset{13}{\text{Ddz-Thr(Bu}^t)}\text{-}\overset{14}{\text{Lys(Z)}}\text{-}\overset{15}{\text{Asp(OBu}^t)}\text{-}\overset{16}{\text{Leu}}\text{-}\overset{17}{\text{LysZ)}}\text{-}\overset{18}{\text{Glu}}\text{-}$$
$$\overset{19}{\text{(OBu}^t)}\text{-Lys(OH) (Z),}$$

wobei der Asparaginsäurerest 15 als Amid oder Alkylamid vorliegen kann, sowie dessen Methylester.

16. Thymosin-$\alpha_1$-Fragment IV der Formel

$$\overset{20}{\text{Ddz-Lys(Z)}}\text{-}\overset{21}{\text{Glu(OBu}^t)}\text{-}\overset{22}{\text{Val}}\text{-}\overset{23}{\text{Val}}\text{-}\overset{24}{\text{Glu(OH)(OBu}^t),}$$

wobei der Glutaminsäurerest 21 als Amid oder Alkylamid vorliegen kann, sowie dessen Methylester.

17. Thymosin-$\alpha_1$-Fragment V der Formel

$$\overset{25}{\text{Ddz-Glu(OBu}^t)}\text{-}\overset{26}{\text{Ala}}\text{-}\overset{27}{\text{Glu(OBu}^t)}\text{-}\overset{28}{\text{Asn(Mbh)(OBzl)}}$$

wobei der Glutaminsäurerest 25 als Amid oder Alkylamid und der Asparaginrest 28 als Diamid oder Dialkylamid vorliegen können, sowie dessen Benzylester.

18. Thymosin-$\alpha_1$-Fragment VI der Formel

$$\overset{20}{\text{Ddz-Lys(Z)}}\text{-}\overset{21}{\text{Glu(OBu}^t)}\text{-}\overset{22}{\text{Val}}\text{-}\overset{23}{\text{Val}}\text{-}\overset{24}{\text{Glu(OBu}^t)}\text{-}\overset{25}{\text{Glu-}}$$
$$\overset{26}{\text{(OBu}^t)}\text{-}\overset{27}{\text{Ala}}\text{-}\overset{28}{\text{Glu(OBu}^t)}\text{-Asn(Mbh)(OBzl),}$$

wobei die Glutaminsäurereste 21 und 25 und/oder der Asparaginrest 28 als Amid oder Alkylamid bzw. als Diamid oder als Dialkylamid vorliegen können.

19. Thymosin-$\alpha_1$-Fragment VII der Formel

$$\overset{13}{\text{Ddz-Thr(Bu}^t)}\text{-}\overset{14}{\text{Lys(Z)}}\text{-}\overset{15}{\text{Asp(OBu}^t)}\text{-}\overset{16}{\text{Leu}}\text{-}\overset{17}{\text{Lys(Z)}}\text{-}\overset{18}{\text{Glu-}}$$
$$\overset{19}{\text{(OBu}^t)}\text{-}\overset{20}{\text{Lys(Z)}}\text{-}\overset{21}{\text{Lys(Z)}}\text{-}\overset{22}{\text{Glu(OBu}^t)}\text{-}\overset{23}{\text{Val}}\text{-}\overset{24}{\text{Val}}\text{-Glu(OBu}^t)\text{-}$$
$$\overset{25}{\text{-Glu(OBu}^t)}\text{-}\overset{26}{\text{Ala}}\text{-}\overset{27}{\text{Glu(OBu}^t)}\text{-}\overset{28}{\text{Asn(Mbh)(OBzl),}}$$

wobei die Glutaminsäurereste 21 und 25, der Asparaginsäurerest 15 und/oder der Asparaginrest 28 als Amid oder Alkylamid bzw. als Diamid oder als Dialkylamid vorliegen können.

20. Thymosin-$\alpha_1$-Fragment VIII der Formel

$$\overset{7}{\text{Ddz-Thr(Bu}^t)}\text{-}\overset{8}{\text{Ser(Bu}^t)}\text{-}\overset{9}{\text{Ser(Bu}^t)}\text{-}\overset{10}{\text{Glu(OBu}^t)}\text{-}\overset{11}{\text{Ile}}\text{-}\overset{12}{\text{Thr-}}$$
$$\overset{13}{\text{(Bu}^t)}\text{-}\overset{14}{\text{Thr(Bu}^t)}\text{-}\overset{15}{\text{Lys(Z)}}\text{-}\overset{16}{\text{Asp(OBu}^t)}\text{-}\overset{17}{\text{Leu}}\text{-}\overset{18}{\text{Lys(Z)}}\text{-Glu-}$$
$$\overset{19}{\text{(OBu}^t)}\text{-}\overset{20}{\text{Lys(Z)}}\text{-}\overset{21}{\text{Lys(Z)}}\text{-}\overset{22}{\text{Glu(OBu}^t)}\text{-}\overset{23}{\text{Val}}\text{-}\overset{24}{\text{Val}}\text{-Glu(OBu}^t)\text{-}$$
$$\overset{25}{\text{-Glu(OBu}^t)}\text{-}\overset{26}{\text{Ala}}\text{-}\overset{27}{\text{Glu(OBu}^t)}\text{-}\overset{28}{\text{Asn(Mbh)(OBzl),}}$$

wobei die Glutaminsäurereste 10, 21 und 25, der Asparaginsäurerest 15 und/oder Asparaginrest 28 als Amid oder Alkylamid bzw. als Diamid oder als Dialkylamid vorliegen können.

**Claims**

1. Process for the preparation of thymosin-$\alpha_1$ or of a derivative thereof in which at least one of the amino acids 10, 15, 21, 25 and 28 is present as amide or alkylamide and/or the acetyl group is replaced by another acyl radical, by preparing a series of peptide fragments containing protective groups, condensations thereof and subsequent splitting off of the protective groups, characterised in that one
   a) condenses the N-terminal unprotected, C-terminal esterified or carrier-bound, C-terminal peptide fragment in at least 1.5 fold less than the stoichiometric amount with the adjacent N-terminal Ddz-protected, C-terminal unprotected peptide fragment in an anhydrous organic solvent with dicyclohexylcarbodiimide and 1-hydroxybenzotriazole,

b) splits off the Ddz-group of the condensed fragment by the addition of trifluoroacetic acid in small stoichiometric excess,

c) neutralises excess acid with an organic base, and

d) condenses the so-obtained N-terminal unprotected, elongated C-terminal fragment with the next appropriate N-terminal Ddz-protected fragment by repetition of steps a), b) and c) until the peptide chain is complete and finally splits off the remaining protective groups in the usual manner, whereby as side protective groups for Asp and Glu tert.-butyl ester groups are used, for Ser and Thr tert.-butyl groups are used, for Lys benzyloxycarbonyl groups are used and for Asn 4,4′-dimethoxybenzhydryl groups are used and the N-terminal acetyl radical is optionally exchanged for another acyl group.

2. Process according to claim 1, characterised in that peptide fragments are used which contain at least one of the glutamic acid residues 10, 21, 25 and/or the aspartic residue 15 and/or the asparagine residue 28 in the form of the amide or alkylamide or, in the case of the asparagine 28, of the diamide.

3. Process according to claim 1 or 2, characterised in that dimethylformamide is used as solvent in step a).

4. Process according to one of claims 1 to 3, characterised in that trifluoroacetic acid in dichloromethane is added in step b).

5. Process according to claim 4, characterised in that a 1 to 5% solution of trifluoroacetic acid is used.

6. Process according to one of the preceding claims, characterised in that the condensation of step a) is carried out at a temperature between 0 and 30°C.

7. Process according to one of the preceding claims, characterised in that the benzyloxycarbonyl protective groups and a C-terminal benzyl ester group are split off by hydrogenolysis in trifluoroethanol.

8. Process according to one of the preceding claims, characterised in that tert.-butyl ester groups are split off with 40 to 60% trifluoroacetic acid in dichloromethane in the presence of anisole.

9. Process according to one of the preceding claims, characterised in that the 4,4′-dimethylbenzhydryl protective group is split off in pure trifluoroacetic acid.

10. Process according to one of the preceding claims, characterised in that neutralisation is carried out in step b) with N-methylmorpholine.

11. Process according to one of the preceding claims, characterised in that the steps a), b) and c) are carried out on the carrier-bound Fragments V, VI, VII, VIII and IX.

12. Polypeptides of the sequence Ser-Asp-Ala-Ala-Val-Asp-Thr-Ser-Ser-Glu-Ile-Thr-Thr-Lys-Asp-Leu-Lys-Glu-Lys-Lys-Glu-Val-Val-Glu-Glu-Ala-Glu-Asn, characterised in that at least one of the amino acids 10, 15, 21, 25 and/or 28 is present as amide or alkylamide and/or amino acid 1 carries an acyl group different from acetyl with up to 6 C atoms, especially an acylglycine radical.

13. Thymosin-$\alpha_1$ Fragment I of the formula:

$$\overset{1}{\text{Ac-Ser}}(\text{Bu}^t)\text{-}\overset{2}{\text{Asp}}(\text{OBu}^t)\text{-}\overset{3}{\text{Ala}}\text{-}\overset{4}{\text{Ala}}\text{-}\overset{5}{\text{Val}}\text{-}\overset{6}{\text{Asp}}(\text{OH})\text{-}(\text{OBu}^t),$$

whereby, instead of the acetyl group, the fragment can carry another acyl group, especially an acylglycine group, as weel as its benzyl ester.

14. Thymosin-$\alpha_1$ Fragment II of the formula:

$$\overset{7}{\text{Ddz-Thr}}(\text{Bu}^t)\text{-}\overset{8}{\text{Ser}}(\text{Bu}^t)\text{-}\overset{9}{\text{Ser}}(\text{Bu}^t)\text{-}\overset{10}{\text{Glu}}(\text{OBu}^t)\text{-}\overset{11}{\text{Ile}}\text{-}\overset{12}{\text{Thr}}(\text{OH})(\text{Bu}^t),$$

whereby the glutamic acid residue 10 can be present as amide or alkylamide, as well as its methyl ester.

15. Thymosin-$\alpha_1$ Fragment III of the formula:

$$\overset{13}{\text{Ddz-Thr}}(\text{Bu}^t)\text{-}\overset{14}{\text{Lys}}(\text{Z})\text{-}\overset{15}{\text{Asp}}(\text{OBu}^t)\text{-}\overset{16}{\text{Leu}}\text{-}\overset{17}{\text{Lys}}(\text{Z})\text{-}\overset{18}{\text{Glu}}(\text{OBu}^t)\text{-}\overset{19}{\text{Lys}}(\text{OH})(\text{Z}),$$

whereby the aspartic acid residue 15 can be present as amide or alkylamide, as well as its methyl ester.

16. Thymosin-$\alpha_1$ Fragment IV of the formula:

$$\overset{20}{\text{Ddz-Lys}}(\text{Z})\text{-}\overset{21}{\text{Glu}}(\text{OBu}^t)\text{-}\overset{22}{\text{Val}}\text{-}\overset{23}{\text{Val}}\text{-}\overset{24}{\text{Glu}}(\text{OH})(\text{OBu}^t),$$

whereby the glutamic acid residue 21 can be present as amide or alkylamide, as well as its methyl ester.

17. Thymosin-$\alpha_1$ Fragment V of the formula:

$$\overset{25}{\text{Ddz-Glu}}(\text{OBu}^t)\text{-}\overset{26}{\text{Ala}}\text{-}\overset{27}{\text{Glu}}(\text{OBu}^t)\text{-}\overset{28}{\text{Asn}}(\text{Mbh})(\text{OBzl})$$

whereby the glutamic acid residue 25 can be present as amide or alkylamide and the asparagine residue 28 as diamide or dialkylamide, as well as its benzyl ester.

18. Thymosin-$\alpha_1$ Fragment VI of the formula:

$$\overset{20}{\text{Ddz-Lys}}(\text{Z})\text{-}\overset{21}{\text{Glu}}(\text{OBu}^t)\text{-}\overset{22}{\text{Val}}\text{-}\overset{23}{\text{Val}}\text{-}\overset{24}{\text{Glu}}(\text{OBu}^t)\text{-}\overset{25}{\text{Glu}}(\text{OBu}^t)\text{-}\overset{26}{\text{Ala}}\text{-}\overset{27}{\text{Glu}}(\text{OBu}^t)\text{-}\overset{28}{\text{Asn}}(\text{Mbh})(\text{OBzl}),$$

whereby the glutamic acid residues 21 and 25 and/or the asparagine residue 28 can be present as amide or alkylamide or as diamide or as dialkylamide, respectively.

19. Thymosin-$\alpha_1$ Fragment VII of the formula:

$$\overset{13}{\text{Ddz-Thr}}(\text{Bu}^t)\text{-}\overset{14}{\text{Lys}}(\text{Z})\text{-}\overset{15}{\text{Asp}}(\text{OBu}^t)\text{-}\overset{16}{\text{Leu}}\text{-}\overset{17}{\text{Lys}}(\text{Z})\text{-}\overset{18}{\text{Glu}}(\text{OBu}^t)\text{-}\overset{19}{\text{Lys}}(\text{Z})\text{-}\overset{20}{\text{Lys}}(\text{Z})\text{-}\overset{21}{\text{Glu}}(\text{OBu}^t)\text{-}\overset{22}{\text{Val}}\text{-}\overset{23}{\text{Val}}\text{-}\overset{24}{\text{Glu}}(\text{OBu}^t)\text{-}\overset{25}{\text{Glu}}(\text{OBu}^t)\text{-}\overset{26}{\text{Ala}}\text{-}\overset{27}{\text{Glu}}(\text{OBu}^t)\text{-}\overset{28}{\text{Asn}}(\text{Mbh})(\text{OBzl}),$$

whereby the glutamic acid residues 21 and 25, the aspartic acid residue 15 and/or the asparagine residue 28 can be present as amide or alkylamide or as diamide or as dialkylamide, respectively.

20. Thymosin-$\alpha_1$ Fragment VIII of the formula:

$$
\begin{array}{c}
\quad\ 7 \qquad 8 \qquad 9 \qquad 10 \qquad\ \ 11\ \ 12 \\
\text{Ddz-Thr(Bu}^t\text{)-Ser(Bu}^t\text{)-Ser(Bu}^t\text{)-Glu(OBu}^t\text{)-Ile-Thr-} \\
\quad\ 13 \qquad 14 \qquad 15 \qquad\ \ 16\ \ 17 \qquad 18 \\
\text{(Bu}^t\text{)-Thr(Bu}^t\text{)-Lys(Z)-Asp(OBu}^t\text{)-Leu-Lys(Z)-Glu-} \\
\quad\ 19 \qquad 20 \qquad 21 \qquad\quad 22\ \ 23\ \ 24 \\
\text{(OBu}^t\text{)-Lys(Z)-Lys(Z)-Glu(OBu}^t\text{)-Val-Val-Glu(OBu}^t\text{)-} \\
\quad 25 \qquad\ \ 26\ \ 27 \qquad\quad 28 \\
\text{-Glu(OBu}^t\text{)-Ala-Glu(OBu}^t\text{)-Asn(Mbh)(OBzl),}
\end{array}
$$

whereby the glutamic acid residues 10, 21 and 25, the aspartic acid residue 15 and/or the asparagine residue 28 can be present as amide or alkylamide or as diamide or as dialkylamide, respectively.

## Revendications

1. Procédé de préparation de la thymosine-$\alpha_1$ ou d'un dérivé de ce composé dans lequel au moins un des aminoacides 10, 15, 21, 25 et 28 est à l'état d'amide ou d'alcoylamide et/ou le groupe acétyle est remplacé par un autre groupe acyle, par préparation d'une série de fragments peptidiques portant des groupes protecteurs, condensation de ceux-ci et élimination subséquente des groupes protecteurs, caractérisé en ce que:

a) on condense le fragment peptidique à C terminal, non protégé sur le N terminal, estérifié sur le C terminal ou fixé sur un support, en défaut d'au moins 1,5 fois par rapport à la quantité stoechiométrique, avec le fragment peptidique voisin, protégé par un groupe Ddz sur le N terminal, non protégé sur le C terminal, dans un solvant organique anhydre, à l'aide de dicyclohexylcarbodiimide et de 1-hydroxybenzotriazole,

b) on élimine le groupe Ddz du fragment condensé par addition d'acide trifluoroacétique en léger axcès par rapport à la quantité stoechiométrique,

c) on neutralise l'excès d'acide à l'aide d'une base organique, et

d) on condense le fragment ainsi obtenu, prolongé sur le C terminal, non protégé sur le N terminal, avec le fragment suivant immédiatement dans chaque cas, protégé par un groupe Ddz sur le N terminal, en répétant les opérations a), b) et c) jusqu'à ce que le chaîne de peptide soit complète et finalement on élimine de manière habituelle les groupes protecteurs restants, en utilisant en tant que groupes protecteurs latéraux pour Asp et Glu des groupes ester tert.-butylique, pour Ser et Thr des groupes tert.-butyle, pour Lys des groupes benzyloxycarbonyle et pour Asn des groupes 4,4'-diméthoxybenzhydryle, et le cas échéant on échange le groupe acétyle sur l'azote terminal contre un autre groupe acyle.

2. Procédé selon la revendication 1, caractérisé en ce que l'on utilise des fragments peptidiques qui contiennent au moins un des restes d'acide glutamique 10, 21, 25 et/ou le reste d'acide aspartique 15 et/ou le reste d'asparagine 28 à l'état d'amide ou d'alcoylamide ou bien, dans le cas de l'asparagine 28, du diamide.

3. Procédé selon la revendication 1 ou 2, caractérisé en ce que l'on utilise comme solvant, dans le stade opératoire a), le diméthylformamide.

4. Procédé selon l'une des revendications 1 à 3, caractérisé en ce que, au stade opératoire b), on ajoute de l'acide trifluoroacétique dans du dichlorométhane.

5. Procédé selon la revendication 4, caractérisé en ce que l'on utilise une solution d'acide trifluoroacétique à une concentration de 1 à 5%.

6. Procédé selon l'une des revendications qui précèdent, caractérisé en ce que la condensation du stade opératoure a) est effectuée à une température entre 0 et 30°C.

7. Procédé selon l'une des revendications qui précèdent, caractérisé en ce que l'on élimine les groupes protecteurs benzyloxycarbonyle et un groupe ester benzylique sur le C terminal par hydrogénolyse dans le trifluoroéthanol.

8. Procédé selon l'une des revendications qui précèdent, caractérisé en ce que l'on élimine le groupe ester tert.-butylique par l'acide trifluoroacétique à 40 à 60% dans le dichlorométhane en présence d'anisole.

9. Procédé selon l'une des revendications qui précèdent, caractérisé en ce que l'on élimine le groupe protecteur 4,4'-diméthylbenzhydryle dans l'acide trifluoroacétique pur.

10. Procédé selon l'une des revendications qui précèdent, caractérisé en ce que, au stade opératoire b), on neutralise par la N-méthylmorpholine.

11. Procédé selon l'une des revendications qui précèdent, caractérisé en ce que les étapes a), b) et c) sont réalisées sur les fragments V, VI, VII, VIII et IX fixés sur des supports.

12. Polypeptides de séquence Ser-Asp-Ala-Ala--Val-Asp-Thr-Ser-Ser-Glu-Ile-Thr-Thr-Lys-Asp-Leu--Lys-Glu-Lys-Lys-Glu-Val-Val-Glu-Glu-Ala-Glu-Asn, caractérisé en ce que l'un au moins des aminoacides 10, 15, 21, 25 et/ou 28 est à l'état d'amide ou d'alcoylamide et/ou l'aminoacide 1 porte un groupe acyle différent du groupe acétyle avec jusqu'à 6 atomes de C, particulier un reste d'acylglycine.

13. Fragment I de la thymosine-$\alpha_1$, de formule

$$
\begin{array}{c}
\quad 1 \qquad\ \ 2 \qquad\quad 3\ \ \ 4\ \ \ 5\ \ \ 6 \\
\text{Ac-Ser(Bu}^t\text{)-Asp(OBu}^t\text{)-Ala-Ala-Val-Asp(OH)(OBu}^t\text{)}
\end{array}
$$

le fragment pouvant porter, à la place du groupe acétyle, un autre groupe acyle, en particulier un groupe acylglycine, et son ester benzylique.

14. Fragment II de la thymosine-$\alpha_1$, de formule

$$
\begin{array}{c}
\quad\ 7 \qquad\ \ 8 \qquad\ \ 9 \qquad\ 10 \qquad\ \ 11\ \ 7 \\
\text{Ddz-Thr(Bu}^t\text{)-Ser(Bu}^t\text{)-Ser(Bu}^t\text{)-Glu(OBu}^t\text{)-Ile-Thr-} \\
\text{(OH)(Bu}^t\text{)}
\end{array}
$$

le reste d'acide glutamique 10 pouvant être à l'état d'amide ou d'alcoylamide, et son ester méthylique.

15. Fragment III de la thymosine-$\alpha_1$, de formule

$$
\begin{array}{c}
\quad\ 13 \qquad\ 14 \qquad 15 \qquad\ \ 16\ \ 17 \qquad 18 \\
\text{Ddz-Thr(Bu}^t\text{)-Lys(Z)-Asp(OBu}^t\text{)-Leu-Lys(Z)-Glu-} \\
\quad\ 19 \\
\text{(OBu}^t\text{)-Lys(OH)(Z)}
\end{array}
$$

le reste d'acide aspartique 15 pouvant être à l'état d'amide ou d'alcoylamide, et son ester méthylique.

16. Fragment IV de la thymosine-$\alpha_1$, de formule

20 21 22 23 24
Ddz-Lys(Z)-Glu(OBu$^t$)-Val-Val-Glu(OH)(OBu$^t$)

le reste d'acide glutamique 21 pouvant être à l'état d'amide ou d'alcoylamide, et son ester méthylique.

17. Fragment V de la thymosine-$\alpha_1$, de formule

25 26 27 28
Ddz-Glu(OBu$^t$)-Ala-Glu(OBu$^t$)-Asn(Mbh)(OBzl)

le reste d'acide glutamique 25 pouvant être à l'état d'amide ou d'alcoylamide, et le reste d'asparagine 28 à l'état de diamide ou de dialcoylamide, et son ester benzylique.

18. Fragment VI de la thymosine-$\alpha_1$, de formule

20 21 22 23 24 25
Ddz-Lys(Z)-Glu(OBu$^t$)-Val-Val-Glu(OBu$^t$)-Glu-
26 27 28
(OBu$^t$)-Ala-Glu(OBu$^t$)-Asn(Mbh)(OBzl)

les restes d'acide glutamique 21 et 25 et/ou le reste d'asparagine 28 pouvant être à l'état d'amide ou d'alcoylamide ou respectivement de diamide ou de dialcoylamide.

19. Fragment VII de la thymosine-$\alpha_1$, de formule

13 14 15 16 17 18
Ddz-Thr(Bu$^t$)-Lys(Z)-Asp(OBu$^t$)-Leu-Lys(Z)-Glu-
19 20 21 22 23 24
(OBu$^t$)-Lys(Z)-Lys(Z)-Glu(OBu$^t$)-Val-Val-Glu(OBu$^t$)-
25 26 27 28
-Glu(OBu$^t$)-Ala-Glu(OBu$^t$)-Asn(Mbh)(OBzl)

les restes d'acides glutamique 21 et 25, le reste d'acide aspartique 15 et/ou le reste d'asparagine 28 pouvant être à l'état d'amide ou alcoylamide ou respectivement à l'état de diamide ou de dialcoylamide.

20. Fragment VIII de la thymosine-$\alpha_1$, de formule

7 8 9 10 11 12
Ddz-Thr(Bu$^t$)-Ser(Bu$^t$)-Ser(Bu$^t$)-Glu(OBu$^t$)-Ile-Thr-
13 14 15 16 17 18
(Bu$^t$)-Thr(Bu$^t$)-Lys(Z)-Asp(OBu$^t$)-Leu-Lys(Z)-Glu-
19 20 21 22 23 24
(OBu$^t$)-Lys(Z)-Lys(Z)-Glu(OBu$^t$)-Val-Val-Glu(OBu$^t$)-
25 26 27 28
-Glu(OBu$^t$)-Ala-Glu(OBu$^t$)-Asn(Mbh)(OBzl

les restes d'acide glutamique 10, 21 et 25, le reste d'acide aspartique 15 et/ou le reste d'asparagine 28 pouvant être présents à l'état d'amide ou d'alcoylamide ou respectivement de diamide ou de dialcoylamide.

1  2  3  4  5  6  7  8  9  10  11  12  13  14  15  16  17  18  19  20  21  22  23  24  25  26  27  28

Ser  Asp.  Ala  Ala  Val  Asp  Thr  Ser  Ser  Glu  Ile  Thr  Thr  Lys  Asp  Leu  Lys  Glu  Lys  Lys  Glu  Val  Val  Glu  Glu  Ala  Glu  Asn

OBu$^t$  **V**  OBu$^t$  Mbh
Ddz ◄————————————► OBzl

Z  OBu$^t$  OBu$^t$
Ddz ◄——————— OH  DCC/HOBt (70%)

Z  OBu$^t$  **IV**  OBu$^t$  OBu$^t$  OBu$^t$  Mbh
Ddz ◄——————————————————► OBzl

Bu$^t$  Z  OBu$^t$  Z  OBu$^t$  Z  **VI**
Ddz ◄————— **III** ———— OH  DCC/HOBt (60%)

Bu$^t$  Z  OBu$^t$  Z  OBu$^t$  Z  Z  OBu$^t$  OBu$^t$  OBu$^t$  OBu$^t$  Mbh
Ddz ◄————————————————————————————► OBzl

Bu$^t$  Bu$^t$  Bu$^t$  OBu$^t$  Bu$^t$  **VII**
Ddz ◄——— **II** ———— OH  DCC/HOBt (67%)

Bu$^t$  Bu$^t$  Bu$^t$  OBu$^t$  Bu$^t$  Bu$^t$  Z  OBu$^t$  Z  OBu$^t$  Z  Z  OBu$^t$  OBu$^t$  OBu$^t$  OBu$^t$  Mbh
Ddz ◄——————————————————————————————————————► OBzl

Bu$^t$  OBu$^t$  OBu$^t$  **VIII**
Ac ◄——— **I** ———— OH  DCC/HOBt (52%)

Bu$^t$  OBu$^t$  OBu$^t$  Bu$^t$  Bu$^t$  Bu$^t$  OBu$^t$  Bu$^t$  Bu$^t$  Z  OBu$^t$  Z  Z  OBu$^t$  OBu$^t$  OBu$^t$  OBu$^t$  Mbh
Ac ◄——————————————————————————————————————— OBzl

1. H$_2$/Pd/TFEtOH  (90%)
2. CF$_3$COOH                **IX**
Ac ◄————————————————————————————————————

Thymosin a$_1$

9